(19) Europäisches Patentamt
European Patent Office
Office européen des brevets

(11) Publication number: 0 493 642 A1

(12) **EUROPEAN PATENT APPLICATION**

(21) Application number: **91100035.4**

(22) Date of filing: **02.01.91**

(51) Int. Cl.[5]: **C07C 335/42**, A61K 31/63, C07D 203/12, C07F 3/02, C07F 1/08, C07C 311/48, C07C 309/76, C07C 311/53, C07C 309/88, C07F 3/10

(43) Date of publication of application: **08.07.92 Bulletin 92/28**

(84) Designated Contracting States: **CH FR GB IT LI SE**

(71) Applicant: **Takayanagi, Takeo, Dr.**
**Ellsworth Avenue, Yonkers**
**New York, N.Y. 10705(US)**

(72) Inventor: **Takayanagi, Takeo, Dr.**
**Ellsworth Avenue, Yonkers**
**New York, N.Y. 10705(US)**

(74) Representative: **Türk, Dietmar, Dr. rer. nat.**
**Türk, Gille + Hrabal Patentanwälte**
**Brucknerstrasse 20**
**W-4000 Düsseldorf 13(DE)**

(54) **Novel sulfonyl derivatives.**

(57) The present invention relates to novel sulfonyl compounds

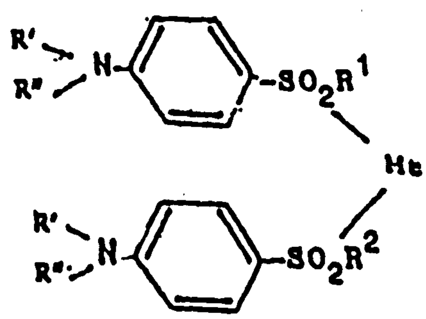

I

wherein R' and R'' are the same or different and are each hydrogen, $-COCHR^4$, $-COC(R^4)_3$ $-SO_2CH_3$, $-COOCH_2CH_3$, $-CH_2-CH_2-R^4$, $-CONH-CH_2CH_2R^4$, $-CONHCH_2COOCH_2CH_3$, $-CH_2CH_2OCONH_2$, and $R_4$ is Cl or a residue selected from

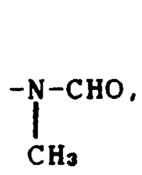

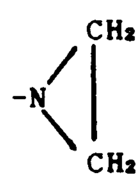

,

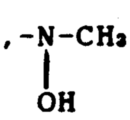

$-NHCOCH_2CH_2OH$, $-NHCOCH_2CH_3$, $-NHOH$, $-NHCH_3$, and

EP 0 493 642 A1

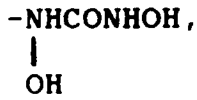

$R^1$ and $R^2$ are the same or different and are each selected from

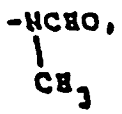, 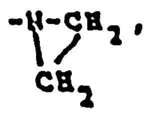, 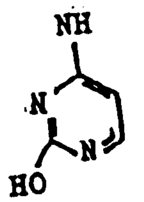, 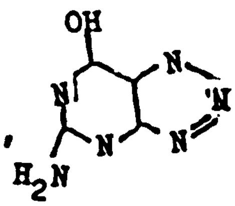

6-mercaptopurinyl, 5-fluorouracily, prednisolyl, salicyl-. hydrazidyl, 1-allyl-2-thiouracilyl, hydroxylamine, isoamidyl, or is an inorganic group consisting of -OH, -SH, -F, -J, $-CCl_3$, or an organic group consisting of $-O-CH_3$, $-O-CH_2CH_3$, $-O-CH=CH_2$, $-O-C_6H_5$ or benzhydrol; or an aminoehtyl residue, or an ether group forming oxonium salts consisting of $ClCH_2OCH_2Cl$, $CH_3OCH_2CH_2OCH_3$, $CH_2ClCH_2OCH_2CH_2Cl$, $C_4H_9OCH_3$, $(C_6H_5)_2O$,

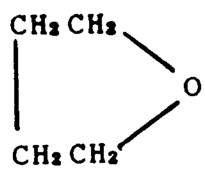

$(CH_2=CHCH_2)_2O$, $CH_2=CHOCH=CH_2$, $C_4H_9OC_4H_9$, $C_6H_5OCH_3$, $CH_3-O-CHCl_2$, $ClCH_2-O-C_2H_5$, $OHCH_2CH_2-O-CH_2CH_2Cl$, $ClCH_2CHCl-C_2H$, and Mt as a metal atom; and wherein the

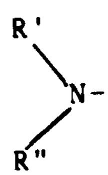

residue can optionally be changed to a linear, branched or cyclic alkyl having 1 to 6 carbon atoms, halogen or a Hg:N-($C_1$-$C_6$) alkyl group.

The invention further relates to a process for the preparation of the novel sulfonyl compounds and derivatives thereof, as well as for a process for manufacturing a pharmaceutical composition containing the novel sulfonyl compound as an active ingredient. Such novel sulfonyl compound are for example capable in the use for inhibitung tissue growth.

The present invention relates to novel sulfonyl compounds and derivatives thereof. The present invention further relates to a process for the preparation of the novel sulfonyl compounds and derivatives thereof,as well as for a process for manufacturing a pharmaceutical composition containing the novel sulfonyl compound as an active ingredient. Such novel sulfonyl compound are for example capable in the use for inhibitung tissue growth.

These and other objects and advantages of the invention will become obvious from the following detailed description.

Subject of the present invention are sulfonyl compounds of the following formula (I)

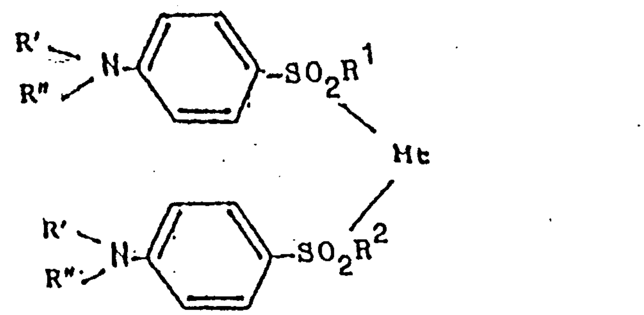

I

wherein R' and R" are the same or different and are each hydrogen, $-COCHR^4$, $-COC(R^4)_3$ $-SO_2CH_3$, $-COOCH_2CH_3$, $-CH_2-CH_2-R^4$, $-CONH-CH_2CH_2R^4$, $-CONHCH_2COOCH_2CH_3$, $-CH_2CH_2OCONH_2$, and $R_4$ is Cl or a residue selected from

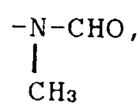

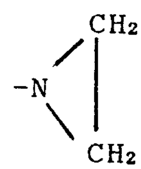

,

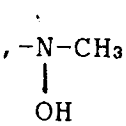

$-NHCOCH_2CH_2OH$, $-NHCOCH_2CH_3$, -NHOH, $-NHCH_3$, and

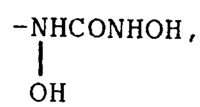

$R^1$ and $R^2$ are the same or different and are each selected from

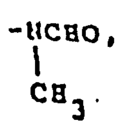

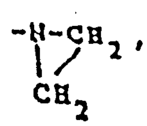

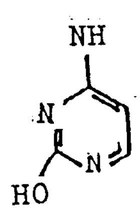

,

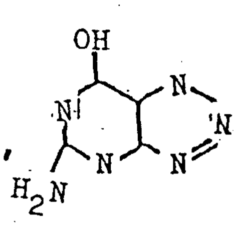

6-mercaptopurinyl, 5-fluorouracily, prednisolyl, salicyl-. hydrazidyl, 1-allyl-2-thiouracilyl, hydroxylamine, isoamidyl, or is an inorganic group consisting of -OH, -SH, -F, -J, $-CCl_3$, or an organic group consisting of $-O-CH_3$, $-O-CH_2CH_3$, $-O-CH=CH_2$, $-O-C_6H_5$ or benzhydrol; or an aminoehtyl residue, or an ether group forming oxonium salts consisting of $ClCH_2OCH_2Cl$, $CH_3OCH_2CH_2OCH_3$, $CH_2ClCH_2OCH_2CH_2Cl$, $C_4H_9OCH_3$, $(C_6H_5)_2O$,

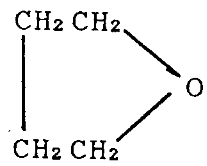

$(CH_2=CHCH_2)_2O$, $CH_2=CHOCH=CH_2$, $C_4H_9OC_4H_9$, $C_6H_5OCH_3$, $CH_3$-O-$CHCl_2$, $ClCH_2$-O-$C_2H_5$, $OHCH_2CH_2$-O-$CH_2CH_2Cl$, $ClCH_2CHCl$-$C_2H$, and Mt as a metal atom; and wherein the

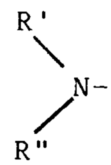

residue can optionally be changed to a linear, branched or cyclic alkyl having 1 to 6 carbon atoms, halogen or a Hg:N-($C_1$-$C_6$)alkyl group.

The metal atom Mt is selected for example from Na, K, Cs, Ca, Mg, Ba, Al, Cu, Ag, Au, Hg, Ni, Fe, Zn, Pd, and Pt The present invention relates further to a process for preparing compounds of the formula I, which comprises condensing of at least one compound of the formula

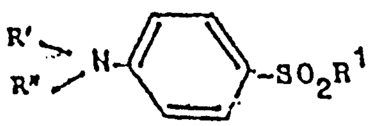

or

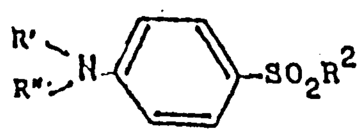

wherein $R^1$, $R^2$, R'and R'' are as defined above and wherein formula (I) can be replaced with or mixed with following formla (III)

$R^5R^6R^7$-$SO_2$-Cl III

wherein $R^5$, $R^6$ and $R^7$ are $C_1$-$C_6$-alkyl, are condensed with a solution of a metal salt of the formula

MtX

wherein Mt is a metal atom selected from Na, K, Cs, Ca, Mg, Ba, Al, Cu, Ag, Au, Hg, Ni, Fe, Pd, and Pt, and X is a residue selected from F, Cl, Br, I, $SO_4$, OH and $CO_3$, in the presence of a coherent solvent to give a compound of the formula I. A suitable coherent solvent is for example.methoxyethanol.

Compounds of the general formula III, $R^5R^6R^7$-$SO_2$-Cl, are for example commercial available sulfonyl chlorides.

The compound of the formula I are useful for preparing inclusion complexes, for example inclusion complexes with pharmaceutical acceptable substances A. The incorporated compounds are selected form the group consisting as well as of liquid as of solid substances.

The sulfonyl radical is acidic and strong charged negativ, on the contrarly the metal salts are charged positiv. At the ion exchanging time of electrophile substitution the interspace of a pair-sulfonyl groups are seemed to be changed into the electric field and occured attractive force in the interspace, then it has been found that several compounds were concomitantly included into the interspace of bifunctional space. In interspace were carried out various interaction on the Donor = Acceptor basis synchronously. Concequently, the formation of solid and steady hybrid compounds acquired.

In this invention is a excellent interspace prepared which could be admitted several compounds at a time.

In the year 1977 Dr. Wulf et al.(Macromol. Chem. 178,2800, (1977))have a interspace in a complex designed and studied the inclusion effect relating bifunctional space on the Donor - Acceptor basis.

Hereby, it is to be emphasized that such a phenomenon doesn't occur in monofunctional case. Regarding the mode or radioactive incorporation of the compounds is schematically illustrated bellow.

(Host and Ouest chemistry)

Tafushi et al.

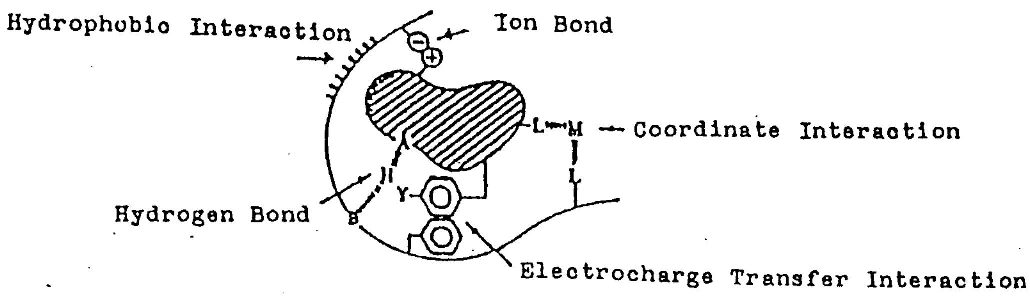


The condensation according to this invention was carried out smoothly in a coherent polar solvent with a metal solution, hereby may be obtained simple metal salts of the formula(I). When in the presence of partnetship( gest component),thereby concomitantly obtained the inclusion compounds. As preferred condensing agents according to this invention are those which are salts of alkaline metal, earthalkaline metal or a transition element, esp-magnesium salt. The inclusion components are particularly irrespective of the state solid or liquid, which are all able to become highly soluble in the water.

Finally it has been found numerous new effective compounds necessary for combination therapie against endless resistant virusses. The new complex compounds of this invention have accurate inhibitory properties against the growth of tissue. But it is now become apparent that the metal fixed compounds could act as a factor of selective destruction of pathogenic organismus, which causes the plasmolyse of resistant virusses.

All the related experiments are carried out since the year 1968 and now under the support of National Cancer Institut of America.

The inclusions complexes have the advantage that the intended effective agents can be brought within the complexes, liquid substances will be purverized, stinking active agents will be deodorized, insoluble material will be easily water soluble and high stability, detoxification of medicine because of included in macromolecule.

Furthermore, it is now substantiated that the present invention has the advantage of obtaining many effective compounds or new substance through adequate variation and combination of the the substituents and the inclusions components.

According to this invention the compounds of the formula (I) may condensed in a polar solvent with a metal salts and if necessary one or more active components reacted.

Accordingly a further object of the present invention is to provide sulfonyl inclusion complexes having pharmaceutical active agent A bounded. The inclusion compounds have the general formula II

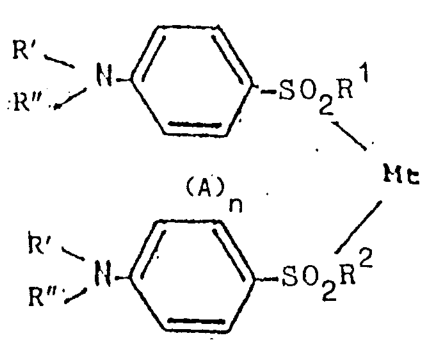

II

wherein $R^1$, $R^2$, R', R'' and Mt are as defined above, A is a pharmaceutical active agent and n is an integer from 1 to 4.

The compounds of the formula II are prepared by mixing at least one compound of the formula

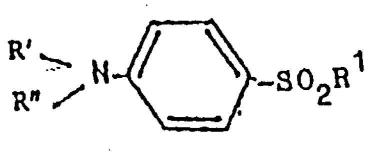

or

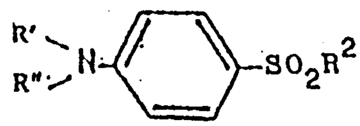

wherein $R^1$, $R^2$, R' and R" are as defined in claim 1 and wherein formula (II) can be replaced with or mixed with following formla (III)

$R^5R^6R^7$-$SO_2$-Cl III

wherein $R^5$, $R^6$ and $R^7$ are $C_1$-$C_6$-alkyl, are condensed with a solution of a metal salt of the formula

MtX

wherein Mt is a metal atom selected from Na, K, Cs, Ca, Mg, Ba, Al, Cu, Ag, Au, Hg, Ni, Fe, Zn, Pd, and Pt, and X is a residue selected from F, Cl, Br, I, OH, $SO_4$, and $CO_3$, with a pharmaceutical active substance A in the presence of a coherent solvent.

A further object of the present invention is to provide a pharmaceutical composition comprising a sulfonyl compound of formula I and/or formula II and pharmaceutically acceptable carrier(s) and/or excipient(s).

The pharmaceutically acceptable carrier and/or excipient may be every useful carrier or excipient.

The pharmaceutical composition of the present invention can be prepared by mixing at least one of the compounds of the formula I or II with pharmaceutically acceptable carrier(s) and/or excipient(s).

Compounds of the pharmaceutically substances A are examplified in the following:

1. Antitumor-agent
Nitromin, Cyclophosphoamid, Thiothepa, 6-Mercaptopurine, 5-Fluorouracil, Prednisolone, Myleran.
2. Antibiotica
Chloramphenicol, Streptmycine, and Penicillin.
3. Curative dyestuff
Pyoctanin, Methylenblue and Aoriflavin.
4. Ferment
Hyaluronidase, Lysozyme Chloride, Collagenase, L-Asparaginase and fixed ferment especially with following metalsalts as: Zn, Cu, Hg, Au, Pd. and Pt.
5. Organic compound
1-Allyl-2-thiounea,Salicylhydracid, N-Methylformamid, Abscisicacid,Phenylchloramine maleate,Quinoneimine,salicylic acid Methylester, Ethylenimine, p-Chloromercuribenzoic Acid,sacharides , glycosides and nicotinic acid.
6. Metalsalt
Metal coupled Amino acid as L-Glutamic Acid, Glycine and Proline.
Metal coupled Amino sugar as: D-Glucosamine, D-Galactosamine and D- Mannosamine.
Metal coupled antimetabolic derivatives as: Pyrimidine or Purine group, besides antimetabolic useful p-Amino benzoic Aid,urethane moreover glycosides. And their applied metal as following: Zn,Cu, Hg, Au,Pd and Pt.
7. Nitroso compound
$(CH_3)_2$N-NO, $(C_2H_5)_2$N-NO, $CH_3$-N-CHO, $Cl_3$C-NO, $(CH_2)_2$N-NO, NO-C(N-$(CH_2)_2)_3$,
8. Hormone
Androgen, Estrogen, Adrenocortical hormone especially Prednisolone, and their metal coupled complexes with following metal salts as: Zn, Cu, Hg, Au, Pd and Pt salts.
9. Ether group

$ClCH_2OCH_2Cl$ $CH_3OCH_2CH_2OCH_3$ $CH_2ClCH_2OCH_2CH_2Cl$ $C_4H_9OCH_3$ $(C_6H_5)_2O$

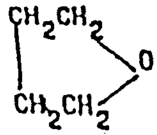

$(CH_2=CHCH_2)_2O$ $CH_2=CHOCH=CH_2$ $C_4H_9OC_4H_9$ $C_6H_5OCH_3$

$CH_3-O-CHCl_2$, $ClCH_2-OC_2H_5$, $OHCH_2CH_2-O-CH_2CH_2Cl$, $ClCH_2CHCl-OC_2H_5$

(may form oxonium salts)

10. Benzoquinone group

2,3,5,6-Tetra(2-chlorethylamino)-1,4-quinone

2,3,5,6-(Tetra(N-methyl-N-formylamino)-1,4-quinone

2,3,5,6-Tetra(N-carbethoxyamino)-1,4-quinone

2,3,5,6-Tetra(N-aziridino)- 1,4-quinone

2,3,5,6-Tetra(N-methyl-N-hydroxylamino)-1,4-quinone

2,3,5,6-Tetra(hydroxyurea)-1,4-benzoquinone

Furthermore, O-benzoquinone, steroides, enzymes, amines, and sod. metals are well applied to this process.

11. Carbamate group.

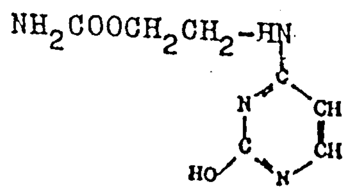

,

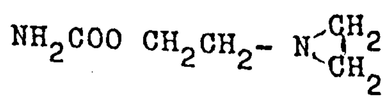

, $NH_2COO\ CH_2C(SH)_3$,

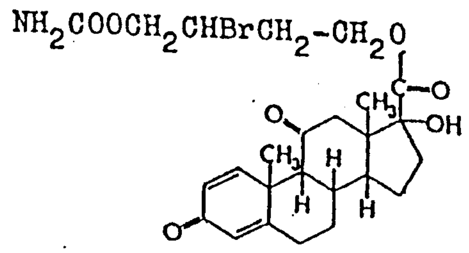

$NH_2COOC_2C(NH_2)_3$,

$NH_2COOCH_2Ø(NHCO-NHOH)_3$, $NH_2COO-NH-CH_3$,

12. Chloralkyl derivatives.

$Cl-CH_2 \cdot CH_2-O-CH_3$; $Cl-CH_2 \cdot CH_2-N(CH_3)-CH_3$

$Cl \cdot CH_2CH_2-NH-CH$,

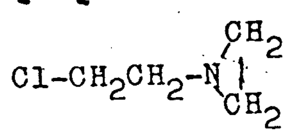

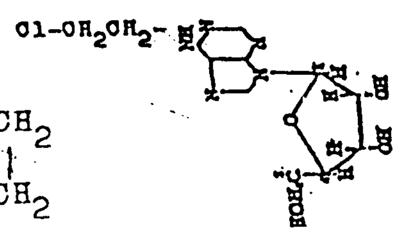

13. Antihistamine Agent

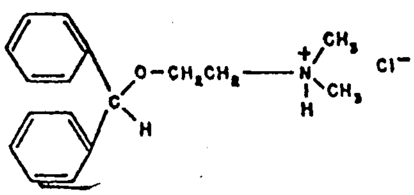 , 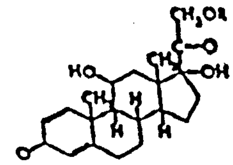

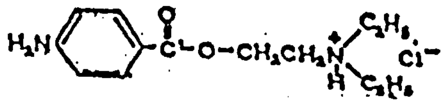 , 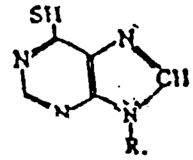 or 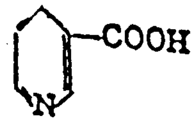

Furthermore, glycoside, pyrimidines, purines, amino acids ferment, hormones or p-amino benzoic acid, isonicotinic acid, amides, and urethane were coupled as polymerisation monomer.

The diphenhydramine is really ether and besides has bifunctional radical therefore, they have strong affinity for salt forming. At present there is no hydramine salt other than dibasic fatty acid salt. This invention relate to the diphenhydramine salts coupled with the almost metabolic relating compounds. The simple hydramine salts are also able to use because of its water solubility and effectiveness. But it has been developed that the above noted salts were condensed to the complex by adding condensing agent such as $MgSO_4$ solution, because of bifunctional affinity of 2-dimethylamino radical which cooporate above noted polymerisation monomer. The resulted complexes can be well used generally. On the other hand the said diphenhydramine salts undergo singly or with monomer sulfamyl metal condensation to obtain more steady and to reinforce the activety of combination therapie. The above noted all salts are the object of the present invention. According to this invention , the preparation of this compounds are accomplished in a solvent, particularly in a ethylene glycol monoethyl ether, which is characteristically causes condensation and moreover dissolves the compounds above mentiond.
The starting compounds of the sulfonyl compounds of the formula(I) will be prepared in the well-Knnown manner from p-acetaminobenzensulfonyl chloride of the formula:

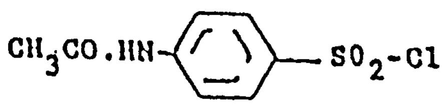 or

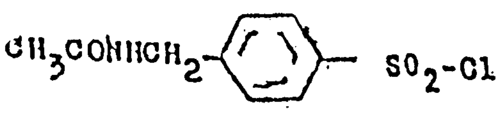

or 4-Carboethoxyamino-benzensulfonyl-chloride of the formula

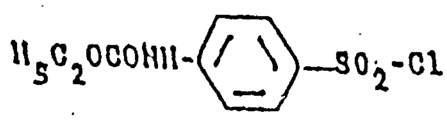

whereby the sulfonyl chloride were prepared from the substituted aromatic compounds and 5 times the amount of chlorosulfonic acid under the cooling at 10 ° C. Then the mixture was heated at 50 ° C several hrs. And the mixture were poured into the icewater. The resulted chloride was dissolved in 30 times pyridine. And 1 mole of substituent $R^1$ was added into the solution and heated for 2 hours at 30 ° C. Then petroleum ether was added until the oily substance separated out. The resulting compound had the following formula

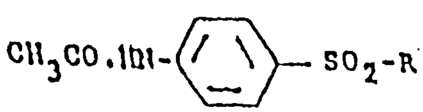

The oily product was added to 5 times of 25 % HCl solution and heated for 20 minutes in a water bath

The hydrolized compound has following formula:

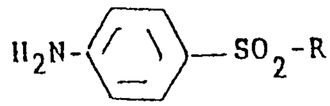

To acetylate the 4-amino group, 1 or 2 molar amounts of the acid chloride were reacted at 10 ° C. When one mole of dichloro or trichloroacetyl chloride were used, the compounds of the following formula were formed

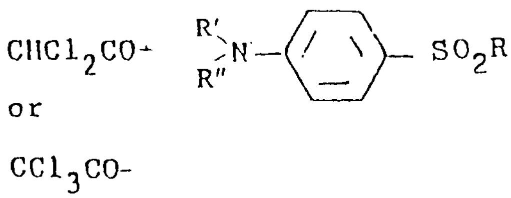

R'R'' = $-COCHCl_2$
$-COCl_3$

When 2 moles of acid chloride were used with heating at 160 to 180 ° C and optionally a catalyst was used, there were formed compounds of the formula

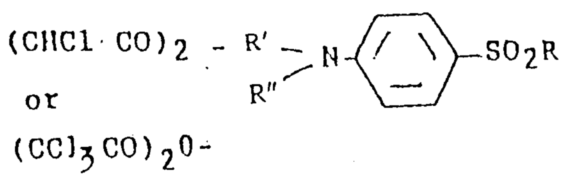

R'R'' = $-(COCHCl)_2$
$-(COCl_3)_2$

Instead of acid chlorides, the acid anhydride or isocyanate could be employed.

By reacting the 4-amino radical with an equimolar amount of 2-chlorethyl isocyanate a compound of the formula was obtained.

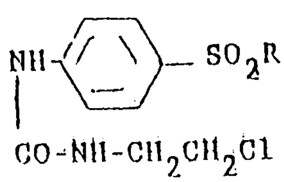

The substitution of above mentioned 4-amino radical with ethylene oxide was carried out in a dioxane solution by adding small amounts of water to obtain a compound of the formula

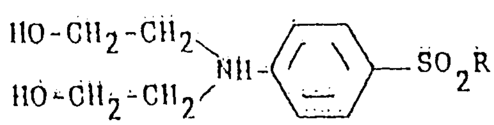

Instead of ethylene oxide, another adequate alkylene oxide can be used.

The obtained oxide substance was dissolved in 20 times of pyridine with small quantities of pyridine and while heating, 2 moles of thionyl chloride were added. The effected mass was isolated to obtain a compound of the formula

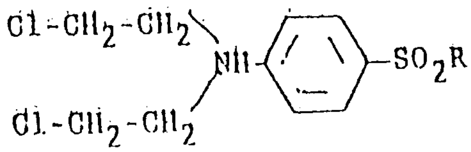

In the following examples there are described several preferred embodiments to illustrate the invention. However, it is to be understood that the invention is not intended to be limited to the specific embodiments.

EXAMPLE 1

2 g of 4-carbethoxyamino-benzene sulfonyl chloride and 1 g of 1-alkyl-2-thiourea were dissolved in 30 ml of pyridine and the mixture was heated to 30 °C and stood overnight. Then, petroleum ether was poured into the mixture until an oil phase began to separate out and the oil phase was dissolved in 30 ml of 2-methoxyethanol. Predisolone carbamate, 6-mercapto-purine, 2-chloroethyl hydroxyamine and fixed hyaluronidase were added to the mixture in a ratio 1:1 and into the mixture were added conc. magnesium solution under vigorous stirring until the solution completely coagulated. The product was washed with petroleum ether and air-dried on filter paper to obtain an easily water-soluble complex which decomposed at 180 °C and has the following formula

Reaction Scheme

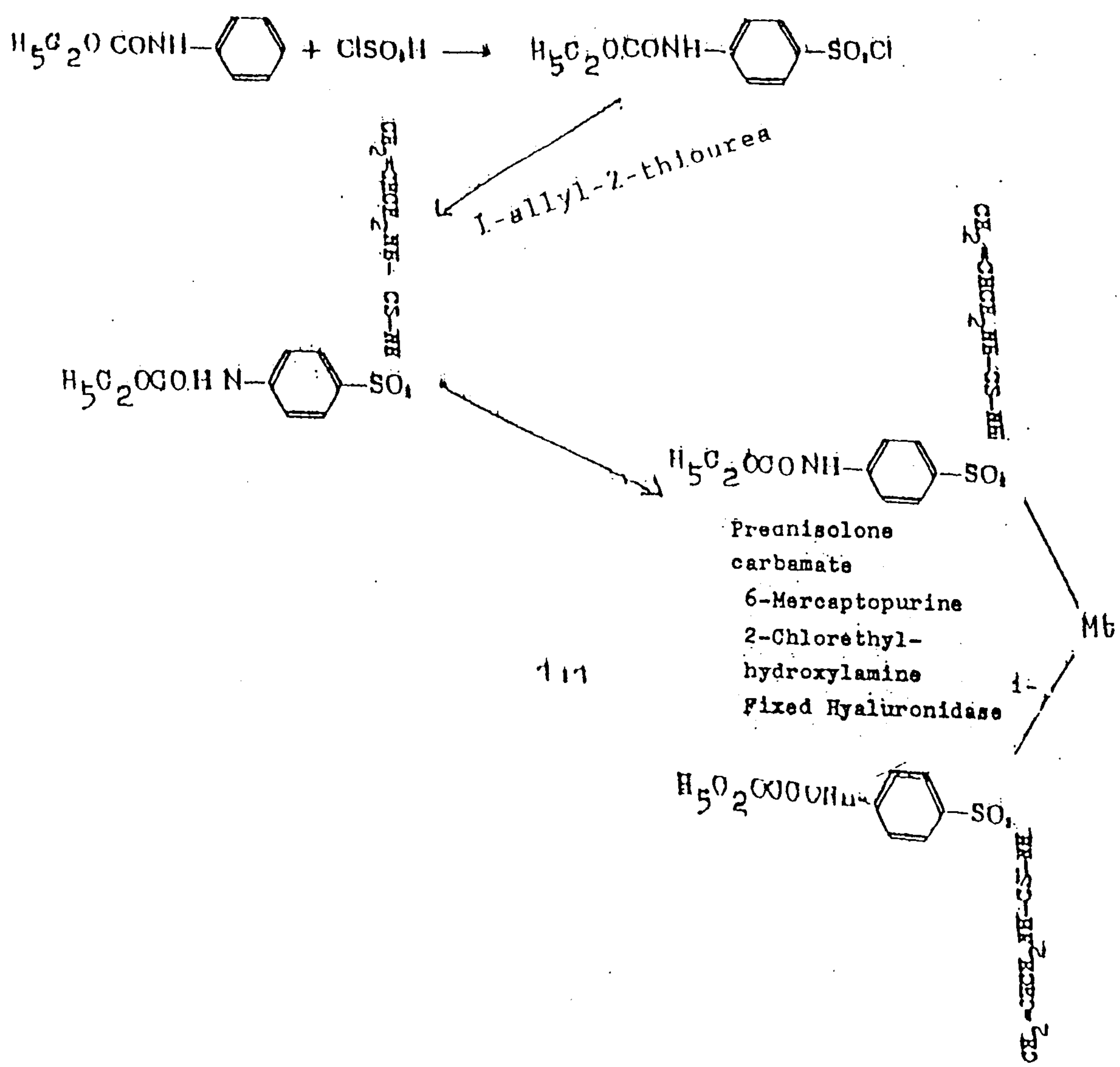


EXAMPLE 2

5 g of 4-carbethoxyamino-benzene sulfonyl chloride and salicyl hydrazide were dissolved in 30 ml of pyridine and the mixture was heated at 30 to 40 ° C for 2 hours. The mixture stood overnight and then petroleum ether was added thereto until an oily phase separated out. The oil was dissolved in 30 ml of 2-methoxyethanol and a mixture of adenosine carbamate, 5-fluorouracil, 2,3,5,6-Tetra-(N-aziridino)-1,4-benzoquinone and Nitroso ethylenimine were added to the mixture in a 1:1 ratio. And it was added with vigorous stirring to a concentrated magnesium sulfate solution until complete coagulation occured. The product was washed with petroleum ether and was dried on filter paper to obtain an extraordinarily water-soluble complex which decomposed at 180 ° C and had the following formula

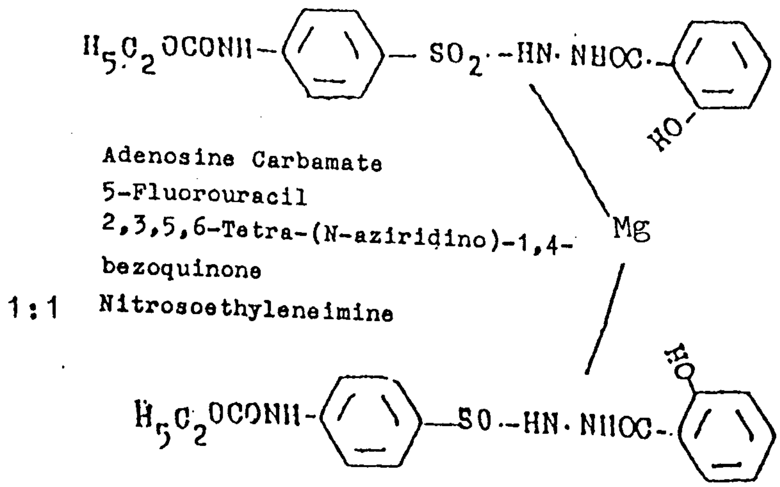

1 : 1

Adenosine Carbamate
5-Fluorouracil
2,3,5,6-Tetra-(N-aziridino)-1,4-
bezoquinone
Nitrosoethyleneimine

EXAMPLE 3

2.5 g of 4-acetamido-benzene sulfonyl chloride and 1 g of ehtylurethane dissolved in 30 ml of pyridine and the mixture was heated at 30° to 40°C for 2 hours and stood overnight. Then petroleum ether was poured into the mixture until an oil phase separated out and the oil phase was dissolved in 30 ml of 25% hydrochloric acid. The mixture was heated on a water bath for 15 minutes to effect hydrolysis and the hydrolyzed product was isolated from the mixture.

The acetylation of the free amino group was effected by dissolving the hydrolyzed product in 30 ml of pyridine and then adding one mole of dichloroacety chloride under cooling. The mixture was poured into ice water and the precipitated product was isolated and dried. The product was dissolved in 30 ml of pyridine and two equivalents of ethyleneimine and the mixture was heated at 30° to 40°C and stood overnight to obtain a viscous mass having the following formula

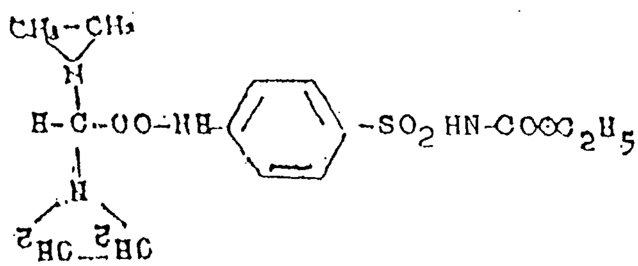

EXAMPLE 3

The viscous mass of Example 3 was dissolved in 40 ml of 2-methoxyethanol and then 2-chloroethyl methyl ether, methyformamid carbamate and $(Cl\ CH_2CH_2)_3C\text{-}NO$ were added in a 1:1 ratio to the mixture. A concentrated magnesium sulfate solution was added to the mixture under stirring until complete coagulation occured. The product was filtered and air-dried on filter paper to obtain an extradordinarily water soluble complex decomposing at 180°C and having the formula

1:1

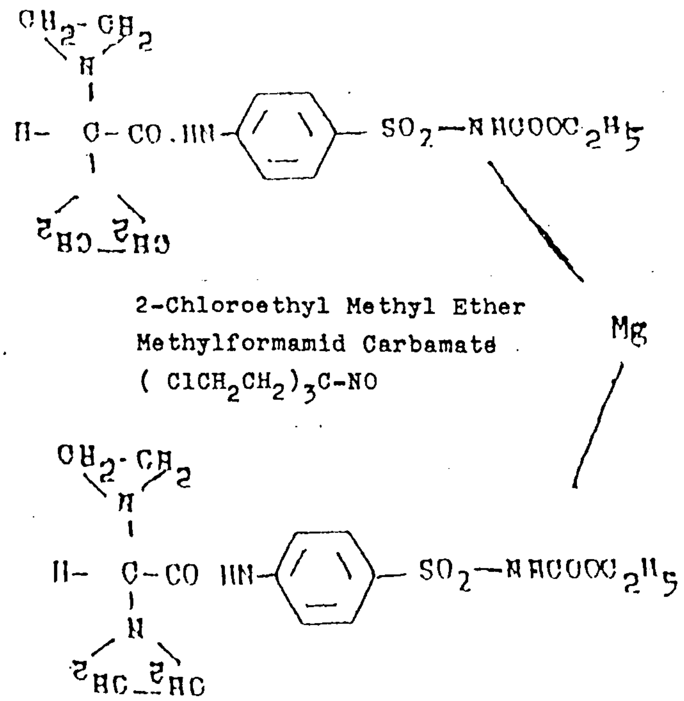


EXAMPLE 4

2.6 g of 4-carboethoxyamino-benzene sulfonyl chloride were dissolved in 40 ml of pyridine and 1.5 ml of monochloromethyl methyl ether were added dropwise under cooling. The mixture stood overnight and then petroleum ether was poured into the mixture to separate out an oil phase. The oil phase was dissolved in 40 ml of 2-methoxyethanol and then a mixture of chloroethyl carbamate, nitrosoethyleneimine and 2-chloroethyl -N-methylformamide were added in a 1:1 ratio. A concentrated magnesium sulfate solution was added dropwise to the mixture until complete coagulation occured. The product was washed with petroleum ether and air-dried on filter paper to obtain an extraordinarily water-soluble complex decomposing at 180 ° C and having the formula

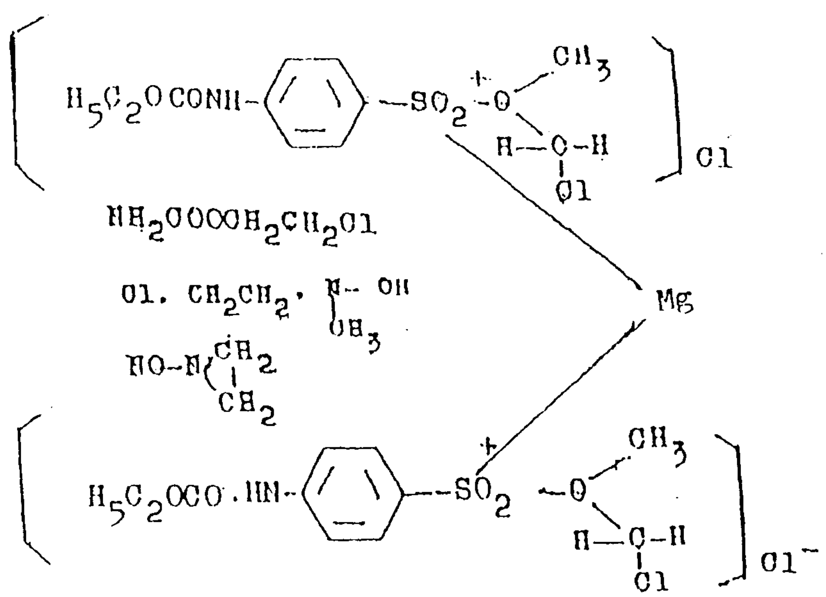

EXAMPLE 5

2.5 g of 4-carbomethoxyamino-benzene sulfonyl chloride were dissolved in 30 ml of pyridine and then 1.5 ml of bis(2-chloroethyl ether were added dropwise to the solution. The mixture stood overnight at room temperature and then petroleum ether was added thereto until an oil phase separated out. The oil layer was dissolved in 40 ml of 2-methoxyethanol and then prednisolone carbamate, nitrosohaloform and Tetra ( N-Methyl-N-Formyl)-amino-o-Quinone were added thereto in the ratio 1:1 A concentrated magnesium sulfate solution was added dropwise thereto under stirring until complete coagulation occured. The resulting complex was highly water-soluble and decomposed at 180 ° C and had the formula

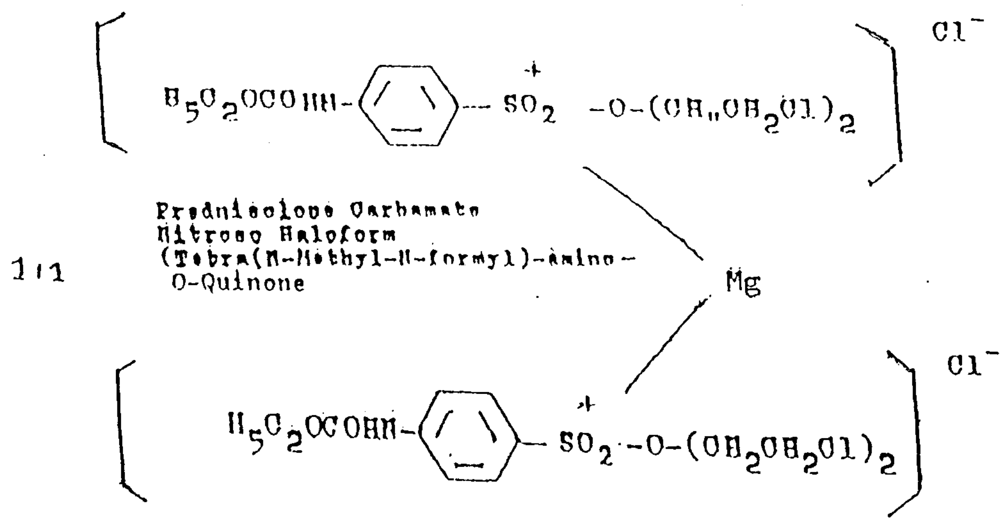


EXAMPLE 6

2.6 g of 4-carboethoxyamino-benzene sulfonyl chloride, 0.5 g of 2-chloroethyl-amine and 0.5 g of ethyl urthane dissolved in 40 ml of pyridine were heated at 30 to 40 ° C for 2 hours and then stood overnight at room temperature. Petroleum ether was added to the mixture until an insoluble oil separated out and the oil was dissolved in 40 ml of 2-methoxyethanol. And a mixture of pheniramine prednisolate, $CH(NHCH_2CH_2Cl)_3$ and Herb extract were added thereto.

Then into the mixture

30% of cupric sulfate solution was added dropwise under stirring until the mixture was completely coagulated. The product was air-dried on filter paper to obtain an easily water-soluble condensation product melting at 180 ° C and having the formula

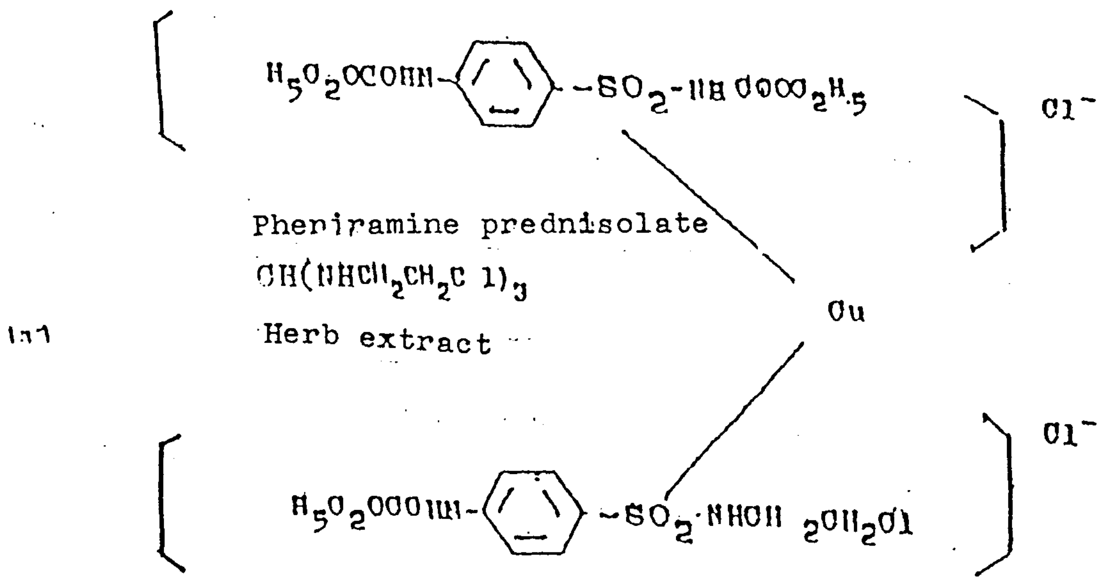


EXAMPLE 7

A mixture of 2 g of 4-carbethoxyamino-benzene sulfonyl chloride, 1 g of potassium iodide and 10 ml of distilled water was heated in a water bath at 50 ° C for 2 hours and was then filtered. The product was air-dried and dissolved in 40 ml of 2-methoxyethanol. A mixture of Pheniramine isonicotinate Nitrosethylenimine, Fixed Pectinase and (Tetra-(2-chloroethyl)-amino)-p-benzoquinone was added to the solution in a ratio of 1:1 under stirring and then conc. 30 or 40 % magnesium sulfate solution was dropweise added untill there was complete coagulation. The product was air-dried on the paper. paper. The resulted compound is easily water soluble, which carbonized with decomposition at 180 ° C and having the formula

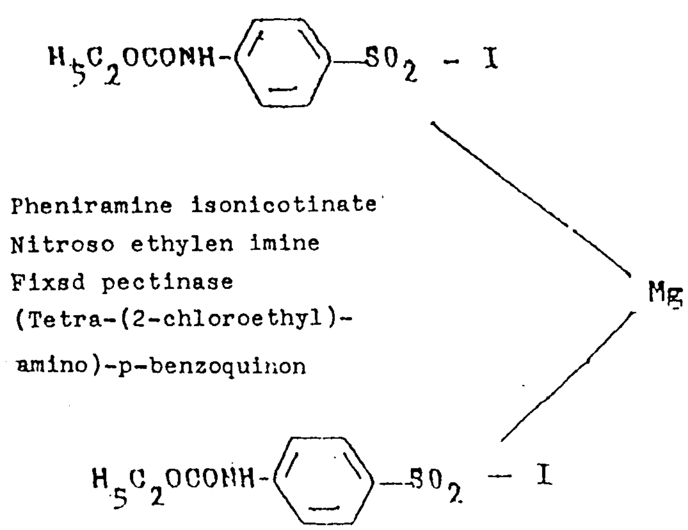


EXAMPLE 8

2g market Sulfisomidine were dissolved in 40ml pyridine and 1g of 2-dimethylaminoethyl chloride were added under cooling. The mixture stood overnight at room temperature and it was allowed to evaporated slowly. The residue was dissolved in 2- methoxyethanol and thereto 5-fluorouracil , 2,3,5,6,-(N-methyl-N-hydroxylamine) -1,4-quinone were added in portion. Then dropwise conc. $MgSO_4$ solution was added untill

complete coagulation occurred. The product was air-dried on filter paper. The resulted product are easily water soluble and carbonized with decomposition at 180 ° C. 180 ° C. The product has following structure formula:

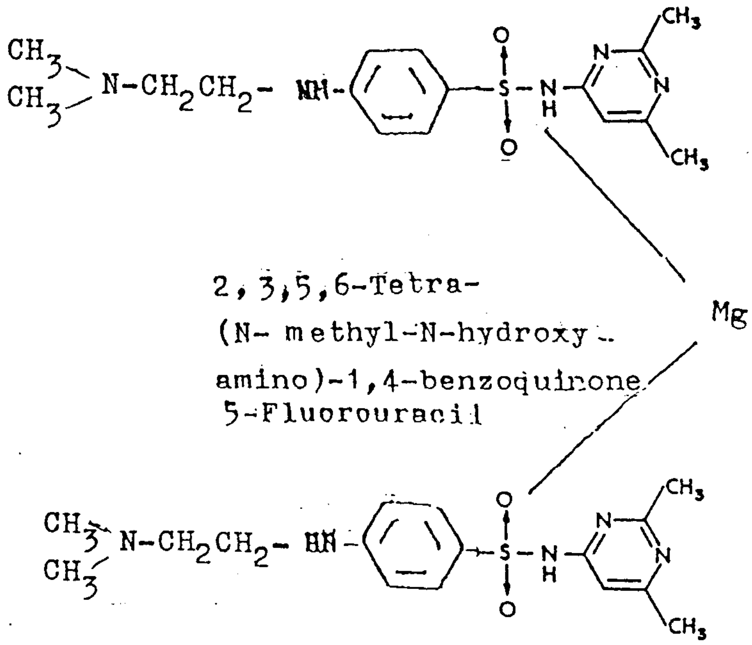

2,3,5,6-Tetra-(N- methyl-N-hydroxy-amino)-1,4-benzoquinone. 5-Fluorouracil

Example 9

2g market Homosulfamine were mixed with an equimolecular weight of HgO and the mixture were slurried with a little water until it coagulated then the resulted friction polymer were dissolved in 40 ml 2-Methoxyethanol.
Into the mixture were at the rate of 1:0.3 glycine coffer. Incidentally, the amino acid is amphoteric and form easily peptide bond also metalbonding.
And then into the mixture were added conc. $MgSO_4$ solution under stirring till the mixture completely coagulated. The resulting product was on the filter paper dried,which is highly in water soluble. And it decomposes at 180 ° C and has following formula:

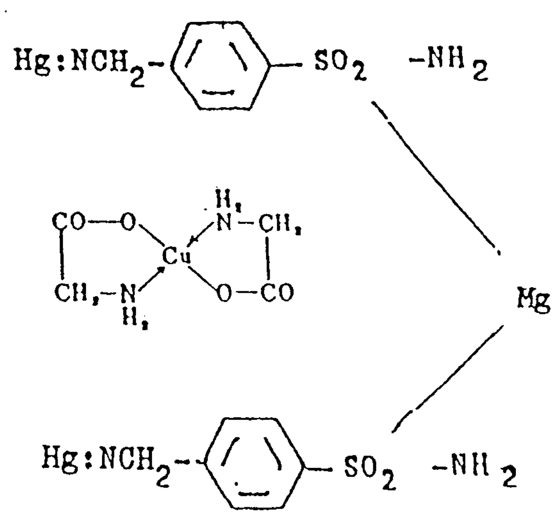

Various modifications of the compositions and method of the invention may be made without departing

from the spirit or scope thereof and it is to be understood that the invention is intended to be limited only as defined in the appended claims.

**Claims**

1. Sulfonyl complex of the following general formula (I)

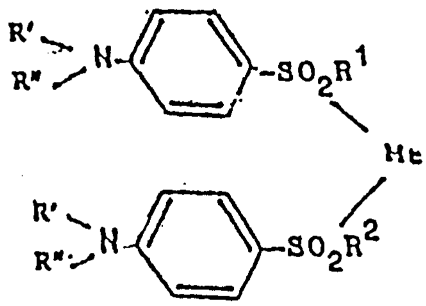

I

wherein R' and R'' are the same or different and are each hydrogen, $-COCHR^4$, $-COC(R^4)_3$ $-SO_2CH_3$, $-COOCH_2CH_3$, $-CH_2-CH_2-R^4$, $-CONH-CH_2CH_2R^4$, $-CONHCH_2COOCH_2CH_3$, $-CH_2CH_2OCONH_2$ , and $R_4$ is Cl or a residue selected from

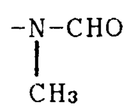 , 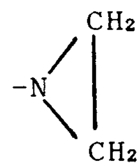 , 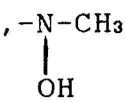

$-NHCOCH_2CH_2OH$, $-NHCOCH_2CH_3$, -NHOH, $-NHCH_3$ , and

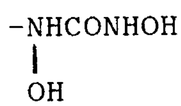 ,

$R^1$ and $R^2$ are the same or different and are each selected from

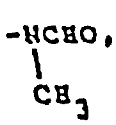 , 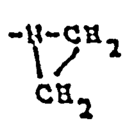 , 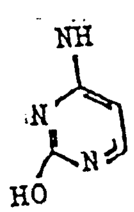 , 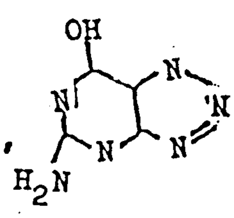

6-mercaptopurinyl, 5-fluorouracily, prednisolyl, salicyl-. hydrazidyl, 1-allyl-2-thiouracilyl, hydroxylamine, isoamidyl, or is an inorganic group consisting of -OH, -SH, -F, -J, $-CCl_3$ , or an organic group consisting of $-O-CH_3$, $-O-CH_2CH_3$, $-O-CH=CH_2$ , $-O-C_6H_5$ or benzhydrol; or an aminoehtyl residue, or an ether group forming oxonium salts consisting of $ClCH_2OCH_2Cl$, $CH_3OCH_2CH_2OCH_3$, $CH_2ClCH_2OCH_2CH_2Cl$, $C_4H_9OCH_3$, $(C_6H_5)_2O$,

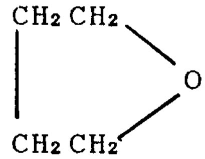

$(CH_2 = CHCH_2)_2O$, $CH_2 = CHOCH = CH_2$, $C_4H_9OC_4H_9$, $C_6H_5OCH_3$, $CH_3$-O-$CHCl_2$, $ClCH_2$-O-$C_2H_5$, $OHCH_2CH_2$-O-$CH_2CH_2Cl$, $ClCH_2CHCl$-$C_2H$, and Mt as a metal atom; and wherein the

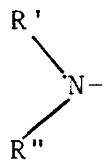

residue can optionally be changed to a linear, branched or cyclic alkyl having 1 to 6 carbon atoms, halogen or a Hg:N-($C_1$-$C_6$)alkyl group.

**2.** Process for preparing sulfonyl complex according to claim 1 characterized in that at least one compound of the formula II

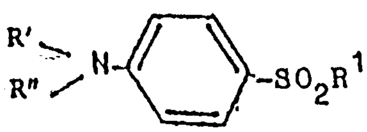

or

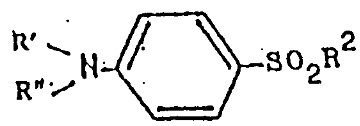

wherein $R^1$, $R^2$, R' and R'' are as defined in claim 1 and wherein formula (I) can be replaced with or mixed with following formla (III)

$R^5R^6R^7$-$SO_2$-Cl III

wherein $R^5$, $R^6$ and $R^7$ are $C_1$-$C_6$-alkyl, are condensed with a solution of a metal salt of the formula

MtX

wherein Mt is a metal atom selected from Na, K, Cs, Ca, Mg, Ba, Cu, Ag, Au, Hg, Ni, Fe, Zn, Pd, and Pt, and X is a residue selected from F, Cl, Br, I, OH, $SO_4$, and $CO_3$, in the presence of a coherent solvent to give a compound of the formula I.

**3.** Pharmaceutical composition containing a compound according to claim 1 and optionally pharmaceutically acceptable carriers and/or excipients.

**4.** Process for preparing a pharmaceutical active composition which comprises mixing a compound according to claim 1 with pharmaceutically acceptable carrier(s) and/or excipient(s).

**5.** Sulfonyl complex of the following general formula (II)

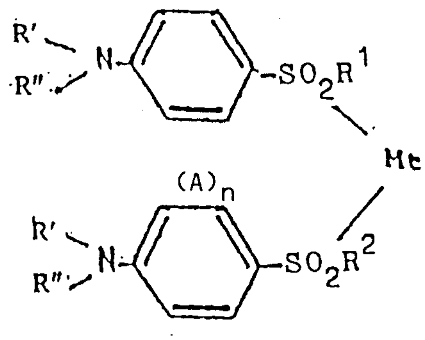

(II)

wherein $R^1$, $R^2$, R', R" and Mt are as defined in claim 1, A is a pharmaceutical active substance and n is an integer from 1 to 4.

**6.** Sulfonyl compound according to claim 5, wherein the pharmaceutical active substance A is an antitumor-agent selected from nitrogen mustard, cyclophosphoamid, thiothepa, 6-mercaptopurine, 5-fluorouracil, vinblastin, L-asparaginase, and myleran.

**7.** Sulfonyl compound according to claim 5, wherein the pharmaceutical active substance A is an antibiotic selected from chloramphenicol, streptomycine or penicilline.

**8.** Sulfonyl complex according to claim 5, wherein the pharmaceutical active substance is a curative dyestuff selected from pyoctanine, methylene blue and acriflavin.

**9.** Sulfonyl compound according to claim 5, wherein the pharmaceutical active substance is a ferment selected from hyaluronidase,lysozyme chloride, collagenase, asparaginase, diastase, pectinase,sulfatase and metal salt fixed ferment.

**10.** Sulfonyl complex according to claim 9, wherein the metal salt is a salt of Zn, Cu, Hg, Au, Pd and Pt.

**11.** Sulfonyl complex according to claim 5, wherein the pharmaceutical active substance A is an organic compound selected from 1-allyl-2-thiourea, salicylhydracid, N-methylformamide,vitamines, abscisic acid, d-maleic acid chlorophenylamine,quinoneimine, salicylic acid methylester, salicylic hydrazide, ethylenimine, p-chloromerxuribenzoic acid, glycosides analoguous, colchitine, nicotinic acid, herb extract, lectine and fixed lectine.

**12.** Sulfonyl complex according to claim 5, wherein the pharmaceutical active substance A is a metal coupled amino acid, metal coupled amino sugar, metal coupled antimetabolic derivative.

**13.** Sulfonyl complex according to claim 12, wherein the metal coupled amino acid is L-glutamic acid, glycine and proline.

**14.** Sulfonyl complex according to claim 12, wherein the metal coupled amino sugar is D-glucosamine, D-galactosamine and D-mannosamine.

**15.** Sulfonyl complex according to claim 12, wherein the metal coupled antimetabolic derivative is a pyrimidine derivative, purine derivative, amino acids, p-amino bonzoic acid derivative, urethanes, and glycosides.

**16.** Sulfonyl complex according to claim 12, wherein the metal salt is a salt of Zn, Cu, Hg, Au, Pd and Pt.

**17.** Sulfonyl complex according to claim 5, wherein the pharmaceutical active substance A is a nitroso sompound selected from $(CH_3)_2N$-NO, $(C_2H_5)_2N$-NO, $CH_3$-N-CHO, $Cl_3C$-NO, $(CH_2)_2N$-NO, NO-C(N-$(CH_2)_2)_3$.

**18.** Sulfonyl complex according to claim 5, wherein the pharmaceutical active substance A is an hormone selected from androgene, estrogene, adrenocortical hormone, prednisolone and metal coupled complexes thereof.

**19.** Sulfonyl complex according to claim 18, wherein the metal salt is a salt of Zn, Cu, Hg, Au, Pd and Pt.

**20.** Sulfonyl complex according to claim 5, wherein the pharmaceutical active substance A is an ether group forming oxonium salts consisting of $ClCH_2OCH_2Cl$, $CH_3OCH_2CH_2OCH_3$, $CH_2ClCH_2OCH_2CH_2Cl$, $C_4H_9OCH_3$, $(C_6H_5)_2O$,

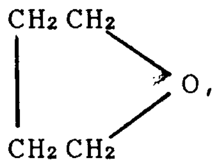

$(CH_2=CHCH_2)$, $CH_2=CHOCH=CH_2$, $C_4H_9OC_4H_9$, $C_6H_5OCH_3$ $CH_2=CHOCH=CH_2$, $C_4H_9OC_4H_9$, $C_6H_5OCH_3$, $CH_3$-O-$CHCl_2$,$ClCH_2$-O-$C_2H_5$, $OHCH_2CH_2$-O-$CH_2CH_2Cl$, $ClCH_2CHCl$-$C_2H_5$.

**21.** Sulfonyl complex according to claim 5, wherein the pharmaceutical active substance A is a metal complex of the following compounds
Benzoquinone group
2,3,5,6-Tetra(2-chlorethylamino)-1,4-quinone
2,3,5,6-Tetra(N-methyl-N-formylamino)-1,4-quinone
2,3,5,6-Tetra(N-carbethoxyamino)-1,4-quinone
2,3,5,6-Tetra(N-aziridino)- 1,4-quinone
2,3,5,6-Tetra(N-methyl-N-hydroxylamino)-1,4-quinone
2,3,5,6-Tetra(hydroxy urea)-1,4-benzoquinone O-benzoquinone,steroides, enzymes, amines, and sod. metals

**22.** Sulfonyl complex according to claim 21, wherein the metal salt is a salt of Zn, Cu, Hg, Au, Pd and Pt.

**23.** Sulfonyl complex according to claim 5, wherein the pharmaceutical active substance A is a chloroalkyl derivative selected from

$Cl-CH_2.CH_2-O-CH_3$, 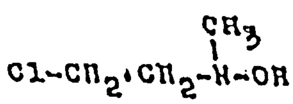

$Cl.CH_2CH_2-HN-OH$,

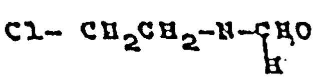

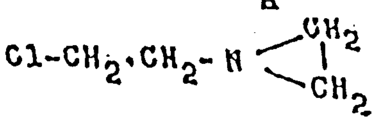

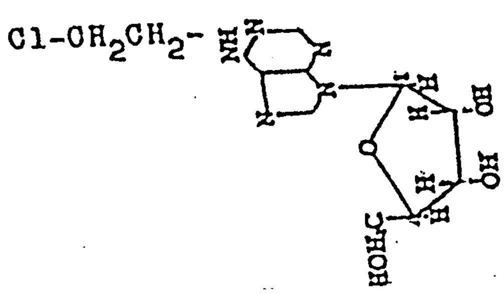

**24.** Sulfonyl complex according to claim 5, wherein the pharmaceutical active substance A is an antihistamine agent selected from

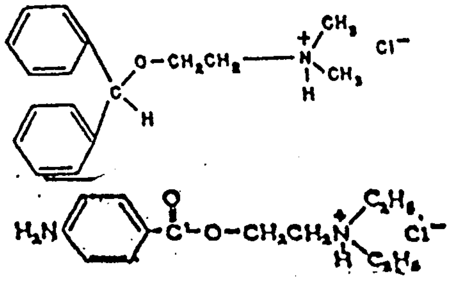

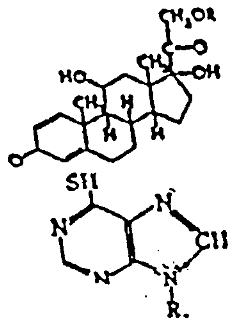

or

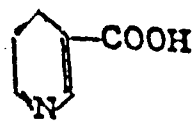

**25.** Sulfonyl complex according to claim 5, wherein the pharmaceutical active substance A is a carbamate selected from

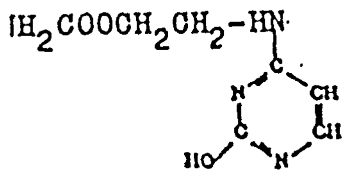

$NH_2COO\ CH_2CH_2-\ N<^{CH_2}_{CH_2}$, $NH_2COO\ CH_2C(SH)_3$,

$NH_2COOCH_2CHBrCH_2-CH_2O$

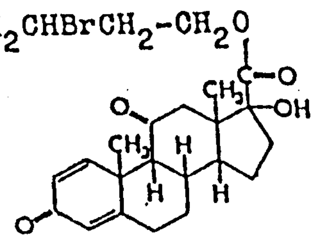

$NH_2COOC_2C(NH_2)_3$,

$COOCH_2C\ (\ NHCO-NHOH)_3$, $NH_2COO-\underset{H}{N}-CH_3$

**26.** Process for preparing a sulfonyl complex according to any of claims 3 to 25 characterized in that at least one compound of the formula

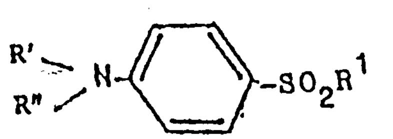

or

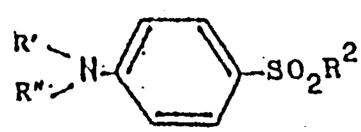

wherein $R^1$, $R^2$, R' and R" are as defined in claim 1 and wherein formula (II) can be replaced with or mixed with following formla (III)

$R^5R^6R^7-SO_2-Cl$ III

wherein $R^5$, $R^6$ and $R^7$ are $C_1$ -$C_6$-alkyl, are condensed with a solution of a metal salt of the formula

MtX

wherein Mt is a metal atom selected from Ca, Mg, Ba, Cu, Ag, Au, Hg, Ni, Fe, Pd, and Pt, and X is a residue selected from F, Cl, Br, I, $SO_4$, and $CO_3$, with a phamaceutical active substance A in the presence of a coherent solvent.

**27.** Process according to claim 26, wherein the solution of the metal salt is an aquous solution of $MgSO_4$.

**28.** Pharmaceutical composition containing compounds according to any of claims 5 to 25 and optionally

pharmaceutically acceptable carriers and/or excipients.

**29.** Process for preparing a pharmaceutical active composition which comprises mixing a compound according to any of claims 5 to 25 with pharmaceutically acceptable carrier(s) and/or excipient(s).

European Patent Office

# EUROPEAN SEARCH REPORT

Application Number

EP 91 10 0035

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 863 910 (T. TAKAYANAGI)<br>* Whole document *<br>--- | 1-29 | C 07 C 335/42<br>A 61 K 31/63<br>C 07 D 203/12<br>C 07 F 3/02<br>C 07 F 1/08<br>C 07 C 311/48<br>C 07 C 309/76<br>C 07 C 311/53<br>C 07 C 309/88<br>C 07 F 3/10 |
| A | EP-A-0 166 615 (ELI LILLY AND CO.)<br>* Pages 1-3; claims *<br>--- | 1-29 | |
| A | US-A-3 823 008 (K. CARPENTER et al.)<br>* Claims *<br>--- | 1,2,5-27 | |
| A | WO-A-8 404 922 (INTERNATIONAL COPPER ASSOCIATION)<br>* Claims *<br>--- | 1-29 | |
| A | CHEMICAL ABSTRACTS, vol. 100, 1984, page 672, abstract no. 131421u, Columbus, Ohio, US; M. CAMALLI et al.: "Structural study on some complexes of silver(I), Ag(t-PP)X, where t-PP = 2,11-bis(di-tert-butylphosphinomethyl)benzo[c]phenanthrene, and X = anionic ligand", & CONGR. NAZ. CHIM. INORG., [ATTI], 15TH 1982, 33-6<br>* Abstract *<br>--- | 1,2,5-27 | **TECHNICAL FIELDS SEARCHED (Int. Cl.5)** |
| A | CHEMICAL ABSTRACTS, vol. 80, 1974, pages 264-265, abstract no. 40985h, Columbus, Ohio, US; Y. KIDANI et al.: "Metal complexes of o- and p-aminobenzenesulfonamides", & YAKUGAKU ZASSHI 1973, 93(10), 1391-3<br>* Abstract *<br>--- | 1,2,5-27 | C 07 C 335/00<br>A 61 K 31/00<br>C 07 D 203/00<br>C 07 F 3/00<br>C 07 F 1/00<br>C 07 C 311/00<br>C 07 C 309/00 |
| E | DE-A-3 921 580 (T. TAKAYANAGI)<br>* Whole document *<br>----- | 1-29 | |

The present search report has been drawn up for all claims

| Place of search | Date of completion of the search | Examiner |
|---|---|---|
| THE HAGUE | 02-09-1991 | SANCHEZ Y GARCIA J.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)